# EUROPÄISCHE PATENTSCHRIFT

(11) **EP 2 812 308 B1**
(45) Veröffentlichungstag und Bekanntmachung des Hinweises auf die Patenterteilung: **20.04.2016**
(21) Anmeldenummer: 13702651.4
(22) Anmeldetag: 07.02.2013
(51) Int. Cl.: C07C 209/36

(54) **ÜBERWACHUNG DES STÖCHIOMETRISCHEN VERHÄLTNISSES BEI DER UMSETZUNG VON NITROAROMATEN MIT WASSERSTOFF**
MONITORING THE STOCHIOMETRIC RATIO WHEN CONVERTING NITROAROMATICS WITH HYDROGEN
SURVEILLANCE DU RAPPORT STÝCHIOMÉTRIQUE LORS DE LA CONVERSION DE NITRO-AROMATES EN HYDROGÈNE

(30) Priorität: 07.02.2012 EP 12154211
(43) Veröffentlichungstag der Anmeldung: 17.12.2014
(73) Patentinhaber: BASF SE, 67056 Ludwigshafen am Rhein (DE)
(72) Erfinder: RAICHLE, Andreas, 67063 Ludwigshafen (DE); HAASE, Stefanie, 01900 Bretnig-Hauswalde (DE); BRAUNSBERG, Holgar, 01968 Senftenberg (DE); BÜTTNER, Johannes, 01945 Ruhland (DE); PENZEL, Ulrich, 01945 Tettau (DE); NETO, Samuel, 68159 Mannheim (DE)
(74) Vertreter: BASF IP Association
(86) Internationale Anmeldenummer: PCT/EP2013/052389
(87) Internationale Veröffentlichungsnummer: WO 2013/117622

(56) Entgegenhaltungen:
- WO-A1-00/35852
- WO-A1-03/068724
- WO-A1-2011/144481

## Beschreibung

Die vorliegende Erfindung betrifft ein kontinuierliches Verfahren zur Herstellung von mindestens einem aromatischen Amin durch Hydrierung von mindestens einem Nitroaromaten mit Wasserstoff, wobei eine mindestens das aromatische Amin enthaltende Flüssigphase und eine mindestens Wasserstoff enthaltende Gasphase vorliegen, in Gegenwart eines in der flüssigen Phase suspendierten Katalysators bei einer Temperatur von 50 bis 250 °C und einem Druck von 5 bis 50 bar, wobei der Druck im Reaktor durch kontinuierliche Anpassung der Menge des dem Reaktor zugeführten Wasserstoffs so gehalten wird, dass er höchstens 10 % um den gewünschten Reaktordruck nach oben oder unten schwankt, die dem Reaktor insgesamt zugeführte Menge an Wasserstoff überwacht wird und die Zufuhr des mindestens einen Nitroaromaten unterbrochen wird, sofern die Wasserstoffaufnahme des Reaktors nicht mindestens 50 Mol.-%, bevorzugt mindestens 70 Mol.-%, besonders bevorzugt mindestens 90 Mol.-%, ganz besonders bevorzugt mindestens 95 Mol.-%, insbesondere mindestens 98 Mol.-%, der für die stöchiometrische Umsetzung des mindestens einen Nitroaromaten zu dem mindestens einen aromatischen Amin benötigten Wasserstoffmenge beträgt.

Verfahren zur Herstellung von aromatischen Aminen durch Hydrierung der entsprechenden Nitroaromaten sind aus dem Stand der Technik bereits bekannt.

WO 02/062729 A2 offenbart ein Verfahren zur Hydrierung von flüssigen, organischen Verbindungen, insbesondere von nitroaromatischen Verbindungen zu den entsprechenden aromatischen Amin-Verbindungen. In einer speziellen Ausführungsform dieses Verfahrens wird offenbart, dass durch Einleiten von Wasserstoff im Reaktor ein bestimmter Druck aufrecht erhalten wird. Weiterhin wird offengelegt, dass die Abgasmenge des Reaktors, in dem die genannte Umsetzung stattfindet, so reguliert, dass die Reinheit des ausgeschleusten Wasserstoffs nach Abtrennung des Wasserdampfes durch Kondensation nur noch 90 Vol-% beträgt.

WO 00/35852 offenbart ebenfalls ein Verfahren zur Herstellung von aromatischen Aminen durch Umsetzen der entsprechenden nitroaromatischen Verbindungen. Auch dieses Dokument offenbart keinen Hinweis darauf, wie eine Schädigung des eingesetzten Katalysators vermieden werden kann.

In der Internationalen Patentanmeldung PCT/EP2011/057427 wird ein Verfahren zur kontinuierlichen Herstellung von Toluylendiamin durch Flüssigphasenhydrierung von Dinitrotoluol mit Wasserstoff in Gegenwart eines suspendierten, Nickel enthaltenden Katalysators, in einem Reaktor, mit einer dem Reaktor nachgeschalteten Produktabtrenneinheit, unter Erhalt eines Produktaustrages aus dem Reaktor umfassend eine Flüssigphase, enthaltend Toluylendiamin und Dinitrotoluol, in der der Nickel enthaltende Katalysator suspendiert ist, beschrieben, wobei die Konzentration an Dinitrotoluol in der Flüssigphase des Produktaustrages aus dem Reaktor im Bereich zwischen dem Reaktor und der nachgeschalteten Produktabtrenneinheit auf einen Wert im Bereich von 1 bis 200 Gew.-ppm, bezogen auf das Gesamtgewicht der Flüssigphase des Produktaustrages aus dem Reaktor, eingestellt wird.

Die genannten Dokumente des Standes der Technik enthalten keinen Hinweis darauf, dass bei einem Verfahren zur Herstellung von aromatischen Aminen aus den entsprechenden Nitroaromaten die Wasserstoffmenge kontrolliert werden soll, und bei einem zu geringen Umsatz bei konstantem Druck die Zufuhr an entsprechenden nitroaromatischen Verbindungen gestoppt werden soll, um eine Schädigung des Katalysators zu vermeiden. Des Weiteren sind die im Stand der Technik genannten Verfahren bezüglich der Schnelligkeit der Detektion einer Störung und der Reaktion auf diese Störung verbesserungsfähig.

Aufgabe der vorliegenden Erfindung gegenüber dem genannten Stand der Technik ist es daher, ein kontinuierliches Verfahren zur Herstellung von aromatischen Aminen durch katalytische Hydrierung der entsprechenden Nitroaromaten bereitzustellen, mit dem zum einen die gewünschten Produkte in hoher Reinheit und Ausbeute zugänglich sind. Eine weitere Aufgabe ist es, ein Verfahren bereitzustellen, mit dem es möglich ist, schon bei geringen Störungen der Reaktion entsprechend handeln zu können, um eine Schädigung des verwendeten Katalysators effizient vermeiden zu können. Dazu ist es notwendig, dass eine auftretende Störung der Reaktion möglichst schnell detektiert werden kann. Des Weiteren soll ein Verfahren bereitgestellt werden, welches das Anspringen/Nicht-Anspringen der katalytischen Hydrierung von Nitroaromaten zu aromatischen Aminen sichtbar macht. Des Weiteren ist es Aufgabe der vorliegenden Erfindung, ein entsprechendes Verfahren bereitzustellen, mit dem es vermieden wird, dass sich nicht umgesetzte nitroaromatische Verbindungen, beispielsweise Dinitrotoluol (DNT), im Reaktor ansammeln, um so eine potentielle Explosionsgefährdung vermeiden zu können.

Diese Aufgaben werden erfüllt durch das erfindungsgemäße kontinuierliche Verfahren zur Herstellung von mindestens einem aromatischen Amin durch Hydrierung von mindestens einem Nitroaromaten mit Wasserstoff, wobei eine mindestens das aromatische Amin enthaltende Flüssigphase und eine mindestens Wasserstoff enthaltende Gasphase vorliegen, in Gegenwart eines in der flüssigen Phase suspendierten Katalysators bei einer Temperatur von 50 bis 250 °C und einem Druck von 5 bis 50 bar, wobei
- der Druck im Reaktor durch kontinuierliche Anpassung der Menge des dem Reaktor zugeführten Wasserstoffs so gehalten wird, dass er höchstens 10 % um den gewünschten Reaktordruck nach oben oder unten schwankt,
- die dem Reaktor insgesamt zugeführte Menge an Wasserstoff überwacht wird und
- die Zufuhr des mindestens einen Nitroaromaten unterbrochen wird, sofern die Wasserstoffaufnahme des Reaktors nicht mindestens 50 Mol.-%, bevorzugt mindestens 70 Mol.-%, besonders bevorzugt mindestens 90 Mol.-%, ganz besonders bevorzugt mindestens 95 Mol.-%, insbesondere mindestens 98 Mol.-%, der für die stöchiometrische Umsetzung des mindestens einen Nitroaromaten zu dem mindestens einen aromatischen Amin benötigten Wasserstoffmenge beträgt.

Das erfindungsgemäße Verfahren zeichnet sich dadurch aus, dass der Druck im Reaktor durch kontinuierliche Anpassung der Menge des dem Reaktor zugeführten Wasserstoffs so gehalten wird, dass er höchstens 10 % um den gewünschten Reaktordruck nach oben oder unten schwankt, die dem Reaktor insgesamt zugeführte Menge an Wasserstoff überwacht wird und die Zufuhr des mindestens einen Nitroaromaten unterbrochen wird, sofern die Wasserstoffaufnahme des Reaktors nicht mindestens 50 Mol-%, bevorzugt mindestens 70 Mol-%, besonders bevorzugt mindestens 90 Mol-%, ganz besonders bevorzugt mindestens 95 Mol-%, insbesondere mindestens 98 Mol-%, der für die stöchiometrische Umsetzung des mindestens einen Nitroaromaten zu dem mindestens einen aromatischen Amin benötigten Wasserstoffmenge beträgt.

Durch das erfindungsgemäße Verfahren kann eine Schädigung des eingesetzten Katalysators durch eine zu hohe Konzentration an Nitroaromaten im Reaktor vermieden werden. Des Weiteren erlaubt die schnelle Unterbrechung der Zufuhr an Nitroaromaten, dass keine zu hohe Nitroaromaten-Konzentration im Reaktor entstehen kann, so dass eine potentielle Explosionsgefährdung vermieden werden kann.

Das erfindungsgemäße Verfahren wird kontinuierlich durchgeführt.

Erfindungsgemäß wird unter "Wasserstoffaufnahme" die dem Reaktor insgesamt zugeführte Wasserstoffmenge abzüglich der ausgeschleusten Wasserstoffmenge verstanden. In einer bevorzugten Ausführungsform ist die ausgeschleuste Wasserstoffmenge so gering, dass sie in erster Näherung vernachlässigt werden kann. Durch die erfindungsgemäße Überwachung der Wasserstoffaufnahme wird eine sehr schnelle Methode zur Detektion des Anspringens/Nichtanspringens bzw. einer ungewollten Verlangsamung ("Einschlafen") der katalytischen Hydrierung von Nitroaromaten zu aromatischen Aminen und mit der erfindungsgemäßen Unterbrechung der Zufuhr des Nitroaromaten eine schnelle Methode zur Vermeidung einer Katalysatordesaktivierung im Falle des Nichtanspringens der Reaktion, z. B. aufgrund einer zu geringen Katalysatormenge oder eines zu inaktiven Katalysators, zur Verfügung gestellt.

In dem erfindungsgemäßen Verfahren wird der Druck im Reaktor, beispielsweise durch kontinuierliche Anpassung der dem Reaktor zugeführten Wasserstoffmenge, im Wesentlichen konstant gehalten. "Im Wesentlichen konstant" bedeutet im Rahmen der vorliegenden Erfindung, dass der Druck im Reaktor höchstens 10%, bevorzugt höchstens 5%, weiter bevorzugt höchstens 3% um den gewünschten Reaktordruck nach oben oder unten schwankt.

Den Druck im Reaktor erfindungsgemäß im Wesentlichen konstant zu halten, gelingt beispielsweise durch einen im Prozessleitsystem (PLS) programmierten PID-(Proportional-Integral-Differential)-Regler.

Um die Wasserstoffaufnahme der erfindungsgemäßen Reaktion von Nitroaromaten mit Wasserstoff bestimmen zu können, wird die insgesamt dem Reaktor zugeführte Menge an Wasserstoff, bevorzugt kontinuierlich, überwacht.

In einer weiteren bevorzugten Ausführungsform wird die Menge und der Gehalt an Wasserstoff in dem ausgeschleusten Strom, überwacht, bevorzugt kontinuierlich. Die ausgeschleuste Wasserstoffmenge ist dabei das Produkt aus gesamter ausgeschleuster Abgasmenge und Wasserstoff-Gehalt. Daher ist es besonders bevorzugt, im Abgasstrom den Wasserstoff-Gehalt zu messen.

In einer besonders bevorzugten Ausführungsform werden die dem Reaktor insgesamt zugeführte Menge Wasserstoff und die ausgeschleuste Menge Wasserstoff überwacht, bevorzugt erfolgen beide Überwachungen kontinuierlich. Da erfindungsgemäß die Zuführung von Wasserstoff an verschiedenen Stellen des Reaktors erfolgen kann, wird erfindungsgemäß bevorzugt unter "insgesamt zugeführte Menge Wasserstoff" die Summe aller zugeführten Mengen Wasserstoff verstanden.

Erfindungsgemäß bevorzugt weist der eingesetzte Wasserstoff eine Reinheit von > 99 Vol.-%, bevorzugt 99,9 - 99,99 Vol.-%, auf. Es kann auch Wasserstoff eingesetzt werden, der inerte Bestandteile enthalten kann, beispielsweise Edelgase und/oder Stickstoff, beispielsweise in einer Menge von 500 bis 2000 ppm Stickstoff oder Argon, siehe beispielsweise WO 02/062729 A2. Bevorzugt ist die Verwendung von Wasserstoff, der besonders wenig Quecksilber enthält, bevorzugt weniger als 30, besonders bevorzugt weniger als 20 und ganz besonders bevorzugt weniger als 2 µg/Nm³. Gegebenenfalls in dem eingesetzten Wasserstoff vorhandene Verunreinigungen werden bei der Bestimmung der Wasserstoffaufnahme erfindungsgemäß bevorzugt berücksichtigt. Ganz besonders bevorzugt wird Wasserstoff eingesetzt, enthaltend mindestens 99,9 Vol-% H₂, maximal 20 Vol.-ppm CO, bevorzugt weniger als 10, besonders bevorzugt weniger als 1 Vol.-ppm CO, maximal 2000 Vol.-ppm Stickstoff, bevorzugt weniger als 100, besonders bevorzugt 10-30 Vol.-ppm Stickstoff, max. 2000 Vol.-ppm Argon, bevorzugt weniger als 200, besonders bevorzugt weniger als 40 Vol.-ppm Argon, max. 25 Vol.-ppm Methan, bevorzugt weniger als 10, besonders bevorzugt weniger als 1 Vol.-ppm Methan, max. 50 Vol.-ppm Sauerstoff, bevorzugt weniger als 30, besonders bevorzugt weniger als 5 Vol.-ppm Sauerstoff, und max. 6000 Vol.-ppm Wasser, bevorzugt weniger als 2500, besonders bevorzugt weniger als 1100 Vol.-ppm Wasser.

Erfindungsgemäß wird bei einer Wasserstoffreinheit von größer 99% diese lediglich gemessen. Bei einer Wasserstoffreinheit von kleiner oder gleich 99% wird diese gemessen und die Zufuhr an Wasserstoff entsprechend eingestellt bzw. die Wasserstoffaufnahme entsprechend bestimmt.

In einer bevorzugten Ausführungsform des erfindungsgemäßen Verfahrens wird die der stöchiometrischen Umsetzung des mindestens einen Nitroaromaten entsprechende Wasserstoffaufnahme des Reaktors spätestens 5 min., bevorzugt spätestens 3 min., nach dem Start der Dosierung des Nitroaromaten erreicht.

Die vorliegende Erfindung betrifft daher bevorzugt das erfindungsgemäße Verfahren, wobei die der stöchiometrischen Umsetzung des mindestens einen Nitroaromaten entsprechende Wasserstoffaufnahme des Reaktors spätestens 5 min., bevorzugt spätestens 3 min., nach dem Start der Dosierung des Nitroaromaten erreicht ist.

Das Unterbrechen der Zufuhr des mindestens einen Nitroaromaten, d. h. eine Außerbetriebnahme des Reaktors, kann erfindungsgemäß nach allen dem Fachmann bekannten Verfahren erfolgen. Beispielsweise kann die Unterbrechung der Zufuhr des Nitroaromaten manuell, beispielsweise durch das die Anlage überwachende Personal oder automatisch beispielsweise durch eine Schaltung im PLS oder hart verdrahtet, erfolgen. Eine Unterbrechung erfolgt erfindungsgemäß bevorzugt bei Vorliegen der oben genannten Bedingungen, d. h. bei Unterschreitung einer definierten Wasserstoffaufnahme oder bevorzugt bei Unterschreitung eines definierten Verhältnisses aus Wasserstoffaufnahme und der für die stöchiometrische Umsetzung des Nitroaromaten zum aromatischen Amin erforderlichen Menge für eine bestimmte Zeit, oder bei Unterschreitung einer Mindestmenge an zugeführtem Wasserstoff.

Es ist erfindungsgemäß bevorzugt, dass die Zufuhr des mindestens einen Nitroaromaten möglichst schnell unterbrochen wird, sofern die erfindungsgemäßen Werte für die Wasserstoffaufnahme unterschritten werden. Bei einer manuellen Unterbrechung liegt die Reaktionszeit beispielsweise bei 1 bis 2 Minuten. Bei einer automatischen Unterbrechung liegt die Reaktionszeit beispielsweise im Sekunden- oder Millisekunden-Bereich.

Erfindungsgemäß erfolgt die Unterbrechung der Zufuhr des mindestens einen Nitroaromaten beispielsweise nach weniger als 2 Minuten, bevorzugt nach weniger als 1 Minute, besonders bevorzugt nach weniger als 30 s, ganz besonders bevorzugt nach weniger als 5 s, jeweils bestimmt von dem Zeitpunkt an, an dem die erfindungsgemäßen Werte für die Wasserstoffaufnahme unterschritten sind.

Um gegebenenfalls auftretende Messwertschwankungen ausgleichen zu können, ist es weiterhin bevorzugt, nach Detektion des Unterschreitens der erfindungsgemäßen Werte für die Wasserstoffaufnahme einen kurzen Zeitraum, beispielsweise 5 bis 30 Sekunden, zu warten, bevor die Zufuhr des mindestens einen Nitroaromaten unterbrochen wird.

Erfindungsgemäß kann die Überwachung der Wasserstoffaufnahme beim Anfahren der Reaktion und während der Reaktion, die kontinuierlich durchgeführt wird, erfolgen.

In einer bevorzugten Ausführungsform des erfindungsgemäßen Verfahrens wird beim Anfahren der Reaktion die Zufuhr des mindestens einen Nitroaromaten nicht unterbrochen, auch wenn die erfindungsgemäßen Werte für die Wasserstoffaufnahme unterschritten werden. In dieser Ausführungsform erfolgt das Unterbrechen der Zufuhr des wenigstens einen Nitroaromaten bei Unterschreiten der erfindungsgemäßen Werte für die Wasserstoffaufnahme beispielsweise erst nach 5 Minuten, bevorzugt nach 4 Minuten, besonders bevorzugt nach 3 Minuten, jeweils nach Beginn des Anfahrens der Reaktion. Dazu wird beispielsweise beim Anfahren die automatische Schaltung manuell überbrückt. Diese geht bevorzugt automatisch nach einer definierten Zeit in den regulären Zustand, d. h. Unterbrechung der Zufuhr des mindestens einen Nitroaromaten beispielsweise nach weniger als 2 Minuten, bevorzugt nach weniger als 1 Minute, besonders bevorzugt nach weniger als 30 s, ganz besonders bevorzugt nach weniger als 5 s, über.

In einer weiteren bevorzugten Ausführungsform wird die Zufuhr des mindestens einen Nitroaromaten nicht mit der gesamten, d. h. 100 Mol.-%, der im Normalbetrieb zugeführten Menge begonnen.

Bevorzugt wird die Zufuhr des mindestens einen Nitroaromaten mit 10 bis 90%, besonders bevorzugt mit 20 bis 80%, ganz besonders bevorzugt mit 30 bis 70 Mol.-% der im Normalbetrieb zugeführten Nitroaromatenmenge begonnen.

Daher betrifft die vorliegende Erfindung auch das erfindungsgemäße Verfahren, wobei die Zufuhr des mindestens einen Nitroaromaten mit 10 bis 90 Mol.-%, bevorzugt 20 bis 80 Mol.-%, besonders bevorzugt 30 bis 70 Mol.-%, der im Normalbetrieb zugeführten Nitroaromatenmenge begonnen wird.

Während der Umsetzung wird die Menge an mindestens einem Nitroaromaten, mit der begonnen wurde, und die erfindungsgemäß bevorzugt weniger als 100 Mol.-% der im Normalbetrieb zugeführten Menge beträgt, auf die im Normalbetrieb zugeführten Menge, d. h. 100 Mol.-%, erhöht. Diese Erhöhung kann erfindungsgemäß durch alle dem Fachmann bekannten Verfahren erfolgen.

Die vorliegende Erfindung betrifft daher bevorzugt das erfindungsgemäße Verfahren, wobei die Nitroaromatenmenge während der Hydrierung schrittweise oder kontinuierlich erhöht wird.

Die erfindungsgemäß bevorzugte Erhöhung kann schrittweise, beispielsweise manuell oder in einer Schrittkette im PLS programmiert, oder kontinuierlich über eine im PLS programmierte Rampe erfolgen.

In einer bevorzugten Ausführungsform des erfindungsgemäßen Verfahrens wird die im Normalbetrieb zugeführte Menge an mindestens einem Nitroaromaten durch schrittweise oder kontinuierliche Zunahme 0,5 bis 120 Stunden, bevorzugt 4 bis 72 Stunden, besonders bevorzugt 8 bis 48 Stunden und ganz besonders bevorzugt 12 bis 24 Stunden nach Start des Verfahrens erreicht.

Die vorliegende Erfindung betrifft daher bevorzugt das erfindungsgemäße Verfahren, wobei die im Normalbetrieb kontinuierlich zugeführte Menge an mindestens einem Nitroaromaten durch schrittweise oder kontinuierliche Zunahme 0,5 bis 120 Stunden nach Start des Verfahrens erreicht wird.

In dem erfindungsgemäßen Verfahren werden die Konzentrationen an Nitroverbindungen bevorzugt auf 1 bis 200 ppm eingestellt.

Die vorliegende Erfindung betrifft daher bevorzugt das erfindungsgemäße Verfahren, wobei die Konzentrationen an Nitroverbindungen bevorzugt auf 1 bis 200 ppm eingestellt werden.

Das erfindungsgemäße Verfahren zur Hydrierung von mindestens einem Nitroaromaten zu mindestens einem aromatischen Amin kann bevorzugt in jedem dem Fachmann als geeignet erscheinenden Lösungsmittel erfolgen. Beispiele für geeignete Lösungsmittel sind ausgewählt aus der Gruppe bestehend aus Wasser, organischen Lösungsmitteln, insbesondere polare organische Lösungsmittel, wie Alkohole, beispielsweise Methanol, Ethanol, Propanol, n-Butanol oder Isobutanol, mindestens einem aromatischen Amin, welches während der erfindungsgemäßen Umsetzung gebildet wird, und Mischungen davon. Erfindungsgemäß als Lösungsmittel weniger bevorzugt sind Ketone, Ester oder Aldehyde.

Die vorliegende Erfindung betrifft daher bevorzugt das erfindungsgemäße Verfahren, wobei ein bevorzugt vorliegendes Lösungsmittel ausgewählt ist aus der Gruppe bestehend aus Wasser, organischen Lösungsmitteln, insbesondere polare organische Lösungsmittel, wie Alkohole, beispielsweise Methanol, Ethanol, Propanol, n-Butanol oder Isobutanol, mindestens einem aromatischen Amin, welches während der erfindungsgemäßen Umsetzung gebildet wird, und Mischungen davon.

Erfindungsgemäß bevorzugt enthält die Flüssigphase im Reaktor vor der Inbetriebnahme mindestens eines der oben genannten Lösungsmittel. In einer bevorzugten Ausführungsform enthält die Flüssigphase im Reaktor vor der Inbetriebnahme im Wesentlichen nur Wasser, bevorzugt Wasser und ein organisches Lösungsmittel, beispielsweise ausgewählt aus der Gruppe bestehend aus Methanol, Ethanol, Propanol, n-Butanol, Isobutanol und Mischungen davon, oder besonders bevorzugt Wasser und ein organisches Lösungsmittel und/oder 1 bis 90 Gew.-%, bevorzugt 10 bis 70 Gew.-%, des mindestens einen aromatischen Amins, welches während der erfindungsgemäßen Umsetzung gebildet wird.

Die vorliegende Erfindung betrifft daher bevorzugt das erfindungsgemäße Verfahren, wobei die Flüssigphase ein Lösungsmittel enthält, ausgewählt aus der Gruppe bestehend aus Wasser, organischen Lösungsmitteln, insbesondere polare organische Lösungsmittel, wie Alkohole, beispielsweise Methanol, Ethanol, Propanol, n-Butanol oder Isobutanol, mindestens einem aromatischen Amin, welches während der erfindungsgemäßen Umsetzung gebildet wird, und Mischungen davon.

In einer bevorzugten Ausführungsform des erfindungsgemäßen Verfahrens wird als Lösungsmittel zu Beginn der Reaktion eine Mischung aus Wasser und dem erfindungsgemäß herzustellenden Amin oder eine Mischung aus Wasser und einem organischen Lösungsmittel, bevorzugt ausgewählt aus der oben genannten Gruppe, eingesetzt. Vorteil dieser bevorzugten Vorgehensweise ist, dass die Schaumbildung der Flüssigphase bei Inbetriebnahme vermindert werden kann, was wiederum eine reibungslosere Inbetriebnahme ermöglicht.

In einer weiteren Ausführungsform des erfindungsgemäßen Verfahrens stammt die Flüssigphase bei der Inbetriebnahme zumindest teilweise aus dem Hydrierbad eines anderen Reaktors, bevorzugt aus einem Reaktor, der für die Umsetzung des gleichen Nitroaromaten mit Wasserstoff zu dem entsprechenden aromatischen Amin verwendet wird, und/oder einem Produktstrom vor, während oder nach der Aufarbeitung eines solchen.

Die vorliegende Erfindung betrifft daher bevorzugt das erfindungsgemäße Verfahren, wobei die Flüssigphase zumindest teilweise aus dem Hydrierbad eines anderen Reaktors, bevorzugt aus einem Reaktor, der auch für die Umsetzung des gleichen Nitroaromaten mit Wasserstoff zu dem entsprechenden aromatischen Amin verwendet wird, und/oder einem Produktstrom vor, während oder nach der Aufarbeitung eines solchen, stammt.

Im Allgemeinen kann das erfindungsgemäße Verfahren bei jeder dem Fachmann für geeignet, insbesondere sicherheitstechnisch geeignet, erscheinenden Konzentration an mindestens einem Nitroaromaten und/oder Wasserstoff durchgeführt werden.

In einer bevorzugten Ausführungsform des erfindungsgemäßen Verfahrens beträgt die Konzentration an Nitrogruppen, d. h. die Summe der Produkte aller enthaltener Nitroaromaten, multipliziert jeweils mit der Anzahl ihrer Nitrogruppen pro Molekül, beispielsweise für die Hydrierung von Dinitrotoluol (DNT) c(Nitroaromaten) = c(DNT) · 2 + c(ANT) · 1 (ANT = Amino-nitro-toluol), oder für die Hydrierung von ortho-Nitrotoluol (o-NT) c(Nitroaromaten) = c(o-NT) · 1, in der Flüssigphase des Produktaustrages aus dem Reaktor im Bereich zwischen dem Reaktor und der bevorzugt nachgeschalteten Produktabtrenneinheit 0 bis 2000 Gew.-ppm, bevorzugt 0,5 bis 1000 Gew.-ppm, besonders bevorzugt 1 bis 200 Gew.-ppm und ganz besonders bevorzugt 1 bis 50 Gew.-ppm, jeweils bezogen auf das Gesamtgewicht der Flüssigphase des Produktaustrages aus dem Reaktor. Dies dient gleichsam einer abschließenden Kontrolle der Misch- und Reaktionsprozesse in den vorgelagerten Reaktoren.

Möglichkeiten, diese Konzentration erfindungsgemäß einzustellen, sind dem Fachmann an sich bekannt.

"Flüssigphase des Produktaustrages im Bereich zwischen dem Reaktor und der bevorzugt nachgeschalteten Produktabtrenneinheit" bedeutet im Rahmen der Erfindung ein Flüssigkeitsstrom oder mehrere Flüssigkeitsströme, über den oder die der Produktaustrag aus dem Reaktor erfolgt. Bevorzugt erfolgt der Produktaustrag an einem Ort geringer DNT-Konzentration, also typischerweise nach der eigentlichen Reaktionszone, über eine Katalysatorabtrenneinheit. Bei Rührkesselreaktoren wird hierfür häufig ein einfacher Überlauf zur Katalysatorabtrenneinheit genutzt. Bei Schlaufenreaktoren bzw. Gasumlaufreaktoren erfolgt der Produktaustrag hingegen meist aus der äußeren Schlaufenströmung.

Die Sicherstellung einer entsprechenden DNT-Konzentration in genau diesem "flüssigen Produktaustrag im Bereich zwischen dem Reaktor und der Produktabtrenneinheit" dient damit gleichsam einer abschließenden Kontrolle der Misch- und Reaktionsprozesse in den vorgelagerten Reaktoren. Auf die technisch sehr viel aufwändigere Möglichkeit der Sicherstellung geeigneter DNT-Konzentrationen an verschiedenen Stellen direkt im Reaktor sei an dieser Stelle explizit hingewiesen. Die einzustellenden Konzentrationsbereiche würden sich in diesem Fall mit zunehmender Nähe zum Ort der DNT-Zudosierung erheblich erhöhen.

Die Produkt- bzw. Katalysatorabtrenneinheit ist allgemein ein Filter (z.B. ein Membranfilter / Querstromfilter), ein statischer Dekanter (z.B. ein Schwerkraftabscheider, häufig ein Lamellenklärer) oder ein dynamischer Dekanter (z.B. eine Zentrifuge oder ein Düsenseparator). Der Katalysator wird von dem Produkt abgetrennt und anschließend (in der Regel als eingedickte Suspension) in den Reaktor zurückgeführt. Besonders bevorzugt erfolgt der Produktaustrag unter Rückhaltung des Katalysators. Das Amin kann danach nach den üblichen und bekannten Verfahren, beispielsweise durch Destillation oder Extraktion, gereinigt werden.

Die analytische Kontrolle der DNT-Konzentration im flüssigen Produktaustrag aus dem Reaktor im Bereich zwischen dem Reaktor und der nachgeschalteten Produktabtrenneinheit auf der Basis einer wiederholten Messung der Dinitrotoluol-Konzentration im flüssigen Produktaustrag aus dem Reaktor wird in einem zeitlichen Abstand von ≤ 24 h durchgeführt. Bevorzugt wird die Messung in einem zeitlichen Abstand von ≤ 12 h, besonders bevorzugt ≤ 4 h und ganz besonders bevorzugt ≤ 1 h fortgesetzt wiederholt. Die zeitlichen Abstände der Überwachung sind dabei so zu wählen, dass rasch auf Veränderungen der DNT-Konzentration im Bereich zwischen Reaktor und Produktabtrenneinheit reagiert werden kann. Die zur Überwachung zu entnehmenden Proben werden üblicherweise vor der Produktabtrenneinheit (beispielsweise bei Verwendung eines Settlers) oder nach der Produktabtrenneinheit (beispielsweise bei der Verwendung eines Filters) entnommen. Die Messung der DNT-Konzentration kann inline, online oder offline erfolgen.

In einer weiteren bevorzugten Ausführungsform des erfindungsgemäßen Verfahrens wird die Wasserstoffkonzentration in der Gasphase des Reaktors c(H₂) kontinuierlich gemessen. In einer weiter bevorzugten Ausführungsform wird die Menge des ausgeschleusten Gases so eingestellt (bzw. geregelt), dass c(H₂), d. h. die Wasserstoffkonzentration in der Gasphase des Reaktors, 80 bis 99 Vol.-%, bevorzugt 85 bis 98 Vol.-%, besonders bevorzugt 90 bis 95 Vol.-%, beträgt.

Die vorliegende Erfindung betrifft daher bevorzugt das erfindungsgemäße Verfahren, wobei die Wasserstoffkonzentration in der Gasphase des Reaktors 80 bis 99 Vol.-%, bevorzugt 85 bis 98 Vol.-%, besonders bevorzugt 90 bis 95 Vol.-%, beträgt.

Für die Hydrierung von Nitroaromaten zu aromatischen Aminen, insbesondere von Dinitrotoluol zu Toluylendiamin, wurde eine Vielzahl von Katalysatoren entwickelt, wobei die Verbesserung von Ausbeute und Selektivität der Umsetzung sowie die Stabilität der Katalysatoren auch bei höheren Reaktionstemperaturen vorrangige Aufgaben bei der Entwicklung neuer Katalysatoren waren.

Als besonders geeignet haben sich Katalysatoren erwiesen, die als Aktivmasse ein oder mehrere Metalle, ausgewählt aus der Gruppe bestehend aus Platin, Palladium, Rhodium, Ruthenium und Mischungen davon und daneben ein oder mehrere weitere Metalle, ausgewählt aus der Gruppe bestehend aus Nickel, Kobalt, Eisen, Zink und Mischungen davon enthalten, und die auf einem Träger aufgebracht ist.

Vorteilhaft sind insbesondere Katalysatoren, die als Aktivmasse Platin und daneben Nickel, oder auch Katalysatoren, die Palladium, Nickel und Eisen oder Katalysatoren, die Palladium, Nickel und Kobalt, enthalten.

Besonders vorteilhaft sind Hydrierkatalysatoren, die Platin und Nickel auf einem Träger in Form einer Legierung mit einem Atomverhältnis von Nickel zu Platin in der Legierung zwischen 30 : 70 und 70 : 30, enthalten. Diese werden erhalten, indem Platin und Nickel bei der Herstellung der Hydrierkatalysatoren in entsprechenden Mengen eingesetzt werden. Das Atomverhältnis kann beispielsweise durch EDXS (Energy Dispersive X-Ray Spectroscopy) bestimmt werden.

Legierungen aus Platin und Nickel mit anderen Atomverhältnissen sind für das erfindungsgemäße Verfahren prinzipiell auch brauchbar, sie führen jedoch, insbesondere bei der Durchführung der Hydrierung bei höheren Temperaturen, zu geringen Ausbeuten an aromatischem Amin.

Der Katalysator enthält zumeist ca. 1 bis 15 nm große, feinkristalline Metallpartikel der Pt-Ni Legierung verteilt auf z. B. Kohlenstoffteilchen. Stellenweise können 1 bis 2 µm große Ni-Pt Teilchenagglomerate oder -aggregate, aber auch vereinzelte reine Ni oder Pt Teilchen auf dem Träger vorkommen. Die Elektronenbeugungslinien der Metallteilchen liegen zwischen denen von Pt und Ni, welche die Legierungsbildung zusätzlich belegt. Die Metallpartikel sind meist polykristallin, und können mit einem hochauflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide, wie z.B. ZrO₂, TiO₂ eingesetzt. Bei Graphit sind die HSAG (High Surface Area Graphite) mit einer Oberfläche von 50 bis 300 m²/g besonders bevorzugt. Besonders bevorzugt sind physisch oder chemisch aktivierte Aktivkohle oder Ruße, wie Acetylenruß.

Weitere geeignete Katalysatoren enthalten als Aktivkomponente Nickel allein oder zusammen mit mindestens einem Metall der I., V., VI. und/oder VIII. Nebengruppe des Periodensystems. Die Katalysatoren können technisch durch Aufbringen von Nickel und gegebenenfalls mindestens einem der oben genannten zusätzlichen Metalle auf einen geeigneten Träger oder durch Co-Fällung hergestellt werden. Gemäß dieser bevorzugten Ausführungsform der Erfindung weist der Katalysator einen Nickelgehalt zwischen 0,1 und 99 Gew.-%, bevorzugt zwischen 1 und 90 Gew.-%, besonders bevorzugt zwischen 25 und 85 Gew.-% und ganz besonders bevorzugt zwischen 60 und 80 Gew.-%, bezogen auf das Gesamtgewicht des Katalysators auf. Vorzugsweise werden als Metalle der I., II., V., VI. und/oder VIII. Nebengruppe des Periodensystems Palladium, Platin, Rhodium, Eisen, Kobalt, Zink, Chrom, Vanadium, Kupfer, Silber oder ein Gemisch aus zwei oder mehreren davon verwendet. Als Trägermaterialien werden vorzugsweise Aktivkohle, Ruß, Graphit, oder oxidische Trägerkomponenten wie Siliziumdioxid, Siliziumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, ZrO₂, HfO₂ und/oder SiO₂, ZrO₂ und/oder SiO₂, ZrO₂, HfO_{2,} verwendet. Geeignete Katalysatoren dieser Ausführungsform sind z. B. in den Druckschriften EP 1 161 297 A1 und EP 1 165 231 A1 beschrieben.

Als Edukt des erfindungsgemäßen Verfahrens werden Nitroaromaten verwendet. In einer bevorzugten Ausführungsform des erfindungsgemäßen Verfahrens werden Mononitroaromaten oder Dinitroaromaten verwendet, besonders bevorzugt werden erfindungsgemäße Dinitroaromaten zu den entsprechenden aromatischen Diaminen hydriert.

Bevorzugte Mononitroaromaten sind beispielsweise ausgewählt aus der Gruppe bestehend aus Nitrobenzol, Nitrotoluol, beispielsweise 2-Nitrotoluol, 3-Nitrotoluol und/oder 4-Nitrotoluol, und Mischungen davon. Erfindungsgemäß kann Nitrobenzol zu Anilin hydriert werden. Des Weiteren kann Nitrotoluol zu den entsprechenden Toluidinen hydriert werden.

Bevorzugte Dinitroaromaten sind beispielsweise ausgewählt aus der Gruppe bestehend aus Dinitrobenzol, beispielsweise 1,2-Dinitrobenzol, 1,3-Dinitrobenzol und/oder 1,4-Dinitrobenzol, Dinitrotoluol und Mischungen davon. Erfindungsgemäß kann Dinitrobenzol zu Diaminobenzol hydriert werden. Des Weiteren kann Dinitrotoluol zu den entsprechenden Toluylendiamin-Isomeren hydriert werden.

Bevorzugt wird erfindungsgemäß Dinitrotoluol (DNT) eingesetzt. Dinitrotoluol ist dem Fachmann an sich bekannt und kann in verschiedenen Isomeren vorliegen, die im Folgenden abgebildet sind

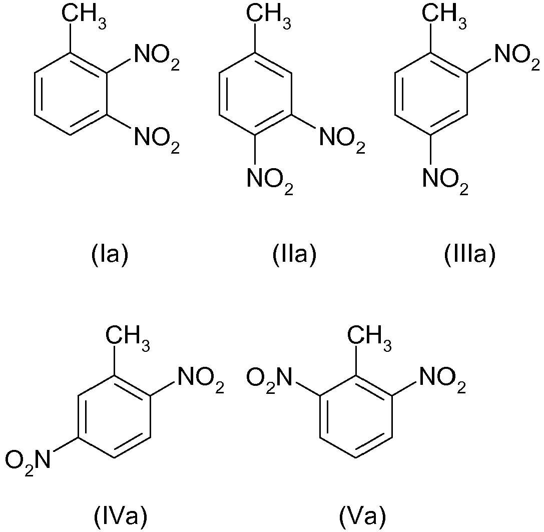

Erfindungsgemäß kann ein einzelnes Isomer ausgewählt aus 2,3-Dinitrotoluol (la), 3,4-Dinitrotoluol (IIa), 2,4-Dinitrotoluol (IIIa), 2,5-Dinitrotoluol (IVa) oder 2,6-Dinitrotoluol (Va) eingesetzt werden. Es ist erfindungsgemäß aber auch möglich, und bevorzugt, eine Mischung enthaltend mindestens zwei der genannten Isomere einzusetzen, beispielsweise eine Mischung enthaltend 2,4-Dinitrotoluol (IIIa) und 2,6-Dinitrotoluol (Va). In einer bevorzugten Ausführungsfrom des erfindungsgemäßen Verfahrens wird eine Mischung von Dinitrotoluol-Isomeren eingesetzt, wie sie bei der zweifachen Nitrierung von Toluol erhalten wird.

In einer bevorzugten Ausführungsform werden als Produkt des erfindungsgemäßen Verfahrens nach Hydrierung die entsprechenden Toluylendiamine erhalten. Die möglichen Isomere sind im Folgenden abgebildet.

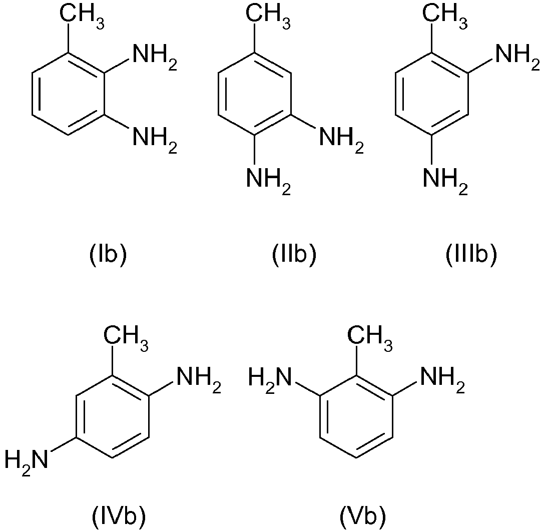

Abhängig davon, welches Isomer bzw. welche Isomere der Dinitrotoluole als Edukte eingesetzt werden, wird als Produkt des erfindungsgemäßen Verfahrens ein entsprechendes Isomer bzw. eine Mischung von mindestens zwei Isomeren des Toluylendiamins erhalten, d. h. erfindungsgemäß bevorzugt findet während der Umsetzung keine Isomerisierung und/oder Hydrierung des aromatischen Systems statt.

Mögliche Produkte sind daher ausgewählt aus Toluylen-2,3-diamin (Ib), Toluylen-3,4-diamin (IIb), Toluylen-2,4-diamin (IIIb), Toluylen-2,5-diamin (IVb) oder Toluylen-2,6-diamin (Vb). Es ist erfindungsgemäß aber auch möglich, und bevorzugt, dass eine Mischung enthaltend mindestens zwei der genannten Isomere erhalten wird, beispielsweise eine Mischung enthaltend Toluylen-2,4-diamin (IIIb) und Toluylen-2,6-diamin (Vb). Des Weiteren ist es möglich, dass in dem erhaltenen Produktgemisch Verbindungen vorliegen, in denen nur eine Nitrogruppe zu der entsprechenden Aminogruppe hydriert worden ist, so dass noch eine Nitrogruppe im Molekül verblieben ist.

Das Verfahren wird bevorzugt bei Raum-Zeit-Ausbeuten von 100 bis 1000 kg · m⁻³ · h⁻¹, bevorzugt 200 bis 600 kg · m⁻³ · h⁻¹, aromatisches Amin durchgeführt. Betrachtet wird dabei das Reaktionsvolumen in m³, wobei dieses dem Gesamtvolumen des Reaktors abzüglich des Volumens von darin vorliegenden Einbauten und des Gasvolumens entspricht.

Das erfindungsgemäße Verfahren wird bevorzugt in einem vertikal stehenden Reaktor durchgeführt. Bevorzugt ist die Länge des Reaktors größer als die Breite. Geeignete Abmessungen sind durch den Fachmann leicht zu bestimmen.

Das erfindungsgemäße Verfahren wird bevorzugt in einem Reaktor durchgeführt, in dem eine interne und eine äußere Schlaufenbewegung des Reaktionsgemisches statt findet. Geeignete Reaktoren sind beispielsweise in der europäischen Patentanmeldung EP 11 158 462.9 oder in der Schrift WO 00/35852 beschrieben.

Hierzu ist in einem bevorzugt vertikal aufrecht stehenden Reaktor an seinem oberen Ende bevorzugt eine Treibstrahldüse angeordnet, die die innere Schlaufenbewegung antreibt, und zwar bevorzugt, indem über die Treibstrahldüse das aus dem Reaktorsumpf abgezogene Reaktionsgemisch, das über eine externe Schlaufe umgepumpt wird, in den oberen Bereich des Reaktors nach unten gerichtet eingedüst wird.

Das über die Treibstrahldüse eingedüste Reaktionsgemisch strömt bevorzugt durch ein zentrales, in Reaktorlängsrichtung angeordnetes Einsteckrohr und durchströmt dieses von oben nach unten. Das zentrale Einsteckrohr kann als einfaches Rohr ausgestaltet sein. Unterhalb des Einsteckrohrs kehrt sich das Reaktionsgemisch in einer internen Schlaufenbewegung außerhalb des Einsteckrohrs um und strömt erneut nach oben.

Zur Strömungsumkehr ist bevorzugt eine Prallplatte unterhalb des Einsteckrohrs angeordnet.

Durch das bevorzugt konzentrisch vorliegende Einsteckrohr wird in Verbindung mit der bevorzugt vorliegenden Prallplatte die Schlaufenströmung innerhalb des Reaktors, d. h. die interne Schlaufenströmung, stabilisiert.

Durch den Treibstrahl wird Gas aus dem Gasraum in die Flüssigkeit in Form von Gas-blasen bis zur Prallplatte mitgerissen. Diese Gasblasen steigen im Ringraum, d. h. zwischen Einsteckrohr und Reaktorwand, des Reaktors wieder auf. Durch diese interne Kreisgasfahrweise wird eine große Gas/Flüssig-Phasengrenzfläche zur Verfügung gestellt.

Im Reaktorinnenraum ist zur Wärmeabführung bevorzugt ein Wärmetauscher angeordnet, durch den Kühlwasser strömt, das einen Teil der Reaktionswärme aufnimmt. Der im Reaktorinnenraum angeordnete Wärmetauscher ist bevorzugt ein Fieldrohr-Wärmetauscher. In einer Ausführungsform ist der im Innenraum des Reaktors angeordnete Wärmetauscher ein Rohrschlangen-Wärmetauscher. In einer weiteren Ausführungsform ist der im Innenraum des Reaktors angeordnete Wärmetauscher ein Rohrbündel-Wärmetauscher. In einer weiteren Ausführungsform ist der im Innenraum des Reaktors angeordnete Wärmetauscher ein Platten-Wärmetauscher.

In einer bevorzugten Ausführungsform des erfindungsgemäßen Verfahrens wird zusätzlich zu dem im Innenraum des Reaktors angeordneten Wärmetauschers ein weiterer Wärmetauscher in der externen Schlaufe eingesetzt. Dieser bevorzugt vorliegende zweite Wärmetauscher dient bevorzugt dazu, den restlichen Teil der Reaktionswärme, der über den internen Wärmetauscher nicht abgeführt werden kann, abzuführen. Bevorzugt kommt dabei ein Rohrbündel-Wärmetauscher zum Einsatz.

Aus der freiwerdenden Reaktionswärme kann Dampf sowohl in dem internen als auch dem externen Wärmetauscher auf zwei Arten erzeugt werden. Zum einen durch Verdampfung eines Teils des Kühlwassers in den Kühlrohren (Direktdampferzeugung) oder durch Erhitzen des Kühlwassers bei einem Druck, der oberhalb des Druckes des zu erzeugenden Dampfes liegt, und anschließende Entspannung auf das Druckniveau des zu erzeugenden Dampfes (Entspannungsverdampfung). Bei dieser Entspannung verdampft ein Teil des Kühlwassers, und das Dampf/WasserGemisch kühlt sich auf die dem Druck des Dampfes entsprechende Siedetemperatur ab.

Beide Arten der Verdampfung können sowohl im internen und als auch externen Wärmetauscher angewendet werden. Ebenso ist eine Kombination beider Verdampfungsarten möglich, das heißt Direktverdampfung im internen Wärmetauscher und Entspannungsverdampfung im externen Wärmetauscher oder umgekehrt.

Abhängig z. B. von der Reaktionstemperatur kann aber auch auf die Dampferzeugung verzichtet und die Wärme durch Rückkühlwasser aufgenommen werden.

Nitroaromaten werden bevorzugt am oberen Ende des Reaktors zugeführt, bevorzugt in die Gasphase oberhalb des Flüssigkeitspegels im Reaktor.

In einer bevorzugten Ausführungsform des erfindungsgemäßen Verfahrens wird die Zuführung des Nitroaromaten durch eine Zulaufleitung und/oder eine Dosiereinrichtung realisiert.

Für das Verfahren ist es bevorzugt, das bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das gewünschte aromatische Amin, Wasser und Katalysator, im Reaktor und/oder im Umpumpkreis erfolgen kann.

Unter einer bestimmungsgemäßen Füllhöhe wird hierbei eine Füllhöhe mit einem Flüssigkeitsspiegel zwischen der Oberkante eines bevorzugt vorliegenden Einsteckrohrs und der Austrittsöffnung der Treibstrahldüse verstanden. Bevorzugt befindet sich der Flüssigkeitsspiegel näher an der Austrittsöffnung der Treibstrahldüse als an der Oberkante des Einsteckrohrs.

In einer bevorzugten Ausgestaltung des erfindungsgemäßen Verfahrens wird das gesamte System aus nichtrostendem Stahl, im Folgenden als Edelstahl bezeichnet, gefertigt, da rostende Stähle, so genannte Schwarzstähle, zur Bildung von Korrosionsprodukten neigen, die bei Verwendung von Membranen als Katalysatorabtrenneinheit zu steigenden Transmembrandrücken führen können.

Bei der erfindungsgemäßen Ausführungsform, dass Nitroaromaten an Nickelkatalysatoren hydriert werden, kann sich die Verwendung von Schwarzstahl als besonders bevorzugt erweisen. Bei dieser im Vergleich zu Edelstahl preiswerteren Variante wirkt die Rostbildung (Eisen(II)-oxid, Eisen(III)-oxid und Kristallwasser) durch die zusätzliche Eisen-promotierende Wirkung positiv auf die Katalyse. In diesem Fall wird als Abtrenneinrichtung beispielsweise ein Schwerkraftabscheider eingesetzt.

In einer bevorzugten Ausführungsform des erfindungsgemäßen Verfahrens wird die Zuführung des Nitroaromaten durch eine Zulaufleitung sowie eine Dosiereinrichtung ohne geometrische Möglichkeit einer Totraumbildung realisiert. Entsprechende Einheiten bzw. Vorrichtungen sind dem Fachmann an sich bekannt. Erfindungsgemäß essentiell ist es dabei, dass bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das aromatische Amin, d. h. das entsprechende Reaktionsprodukt, Wasser und Katalysator, im Reaktor und/oder im Umpumpkreis erfolgen kann. Dies wird erfindungsgemäß beispielsweise dadurch realisiert, dass der Abstand zwischen der Zuführung des Nitroaromaten und der flüssigen Phase beispielsweise 0,01 bis 3 m, bevorzugt 0,05 bis 1,0 m beträgt.

In einer weiter bevorzugten Ausführungsform des erfindungsgemäßen Verfahrens wird die Zuführung des Nitroaromaten über eine oder mehrere unabhängige Rohrleitungen realisiert, deren teilweise oder vollständige Verblockung messtechnisch erfasst werden kann. Entsprechende Rohrleitungen sind dem Fachmann an sich bekannt. Die bevorzugt eingesetzten Rohrleitungen sind beispielsweise aus Metall, beispielsweise Schwarzstahl oder Edelstahl, gefertigt. Die Rohrleitungen weisen bevorzugt Durchmesser auf, die sich nach den Mengen an Nitroaromaten, welche zugeführt werden, bemessen.

Im Allgemeinen können die bevorzugt eingesetzten Rohrleitungen jeden geeigneten Querschnitt aufweisen. In einer bevorzugten Ausführungsform des erfindungsgemäßen Verfahrens weist der Austrittsquerschnitt der Zuführung des Nitroaromaten an den Enden der einen oder mehreren unabhängigen Rohrleitung(en) eine Verengung auf. Der austretende Nitroaromat-Strahl kann laminares oder turbulentes Verhalten aufweisen.

Es ist erfindungsgemäß auch günstig, wenn bei der Einmischung des frisch zugeführten Nitroaromaten in die Reaktionsmischung signifikante lokale Überkonzentrationen vermieden werden. Erhöhte (lokale) Konzentrationen von Nitroaromaten führen zu verstärkter Nebenproduktbildung und Deaktivierung des suspendierten Katalysators. Daraus resultieren ein Ausbeuteverlust und damit ein höherer Aufwand zur Produktaufarbeitung einerseits und erhöhte Katalysatorkosten sowie Kapazitätseinschränkungen andererseits. Außerdem können Sicherheitsprobleme auftreten.

Durch den Treibstrahl des äußeren Wälzkreises wird im Innern des Reaktors bevorzugt eine zirkulierende Strömung hervorgerufen. Der Abwärtsstrombereich dieser Zirkulationsströmung ist durch die Querschnittsfläche des bevorzugt vorliegenden Einsteckrohres parallel zur Reaktorachse festgelegt. Der Aufwärtsströmbereich wird durch die Querschnittsfläche des Ringspalts zwischen dem bevorzugt vorliegenden Einsteckrohr und Reaktormantel festgelegt.

Der Treibstrahl des äußeren Wälzkreises breitet sich im Reaktionsmedium bevorzugt als turbulenter Freistrahl aus. Am Umfang eines turbulenten Freistrahls findet durch Impulseintrag eine Beschleunigung und Einmischung des umgebenden Fluids statt.

Bei Aufbringung von Nitroaromaten auf die Flüssigkeitsoberfläche im Reaktorinneren wird die Wirksamkeit der Einmischung demzufolge mit zunehmender Nähe zum Auftreffbereich des Treibstrahls auf die Flüssigkeitsoberfläche verbessert. Die Wirksamkeit der Einmischung bestimmt die räumliche Ausdehnung der Einmischzone sowie der Höhe der darin stationär auftretenden Nitroaromat-Überkonzentration im Vergleich zum Gesamtreaktorvolumen.

In der erfindungsgemäß bevorzugten Ausführungsform, in der der erfindungsgemäß eingesetzte Reaktor ein zentrales Einsteckrohr, welches in Reaktorlängsrichtung angeordnet ist, aufweist, wird in dem erfindungsgemäßen Verfahren ein laminarer oder turbulenter Nitroaromaten-Strahl aus einer oder mehreren der Zuführungen des Nitroaromaten im Bereich der auf die Flüssigkeitsoberfläche projizierten Querschnittsfläche des Einsteckrohres, bevorzugt möglichst nahe am Treibstrahl der Düse des äußeren Umlaufs, besonders bevorzugt direkt am Umfang des Treibstrahls der Düse des äußeren Umlaufs auf die Oberfläche der flüssigen Phase, aufgegeben.

In einer weiteren bevorzugten Ausführungsform des erfindungsgemäßen Verfahrens sind die Austrittsöffnungen der einen oder mehreren Zuführungen des Nitroaromaten zur Erzeugung eines radialen Impulses um bis zu 90° in Richtung der Symmetrieachse des Reaktors geneigt. In dieser Ausführungsform sind die Austrittsöffnungen um beispielsweise 10 bis 80°, bevorzugt 10 bis 45° in Richtung der Symmetrieachse des Reaktors geneigt.

Das erfindungsgemäße Verfahren weist bevorzugt das Merkmal auf, dass nach einem nichtbestimmungsgemäßen Kontakt zwischen der Zuführung des Nitroaromaten und der Flüssigphase des Reaktors eine teilweise oder vollständige Verblockung der Zuführung des Nitroaromaten messtechnisch erfasst werden kann.

Messtechnische Verfahren und Vorrichtungen zur Erfassung einer entsprechenden Verblockung sind dem Fachmann an sich bekannt, beispielsweise Manometer oder Durchflussmesser. Beispielsweise kann eine Verblockung dadurch detektiert werden, dass ein Stoffstrom bei konstanter Druckdifferenz abnimmt. Des Weiteren kann eine Verblockung beispielsweise durch einen Anstieg des Druckes an der entsprechenden Stelle bei konstantem Stoffstrom detektiert werden.

Der für die Hydrierung notwendige Wasserstoff wird am unteren Ende des Reaktors, bevorzugt über einen Ringverteiler, zugeführt.

Ein weiteres bevorzugtes Merkmal des erfindungsgemäßen Verfahrens ist, dass die Konzentration an Wasserstoff im Reaktionsgemisch, das aus dem Reaktorsumpf in die externe Schlaufe strömt 0,1 Vol.-%, bevorzugt 3 Vol.-%, nicht unterschreitet. Durch diese erfindungsgemäße Mindestkonzentration im Reaktionsgemisch wird beispielsweise gewährleistet, dass ein gewünschter hoher Umsatz des Nitroaromaten, bevorzugt des Dinitrotoluols, und somit eine niedrige Nitroaromat-Konzentration, bevorzugt Dinitrotoluol-Konzentration, auch im Bereich der externen Umlaufströmung erreicht werden.

In einer weiteren bevorzugten Ausführungsform des erfindungsgemäßen Verfahrens wird dem in der externen Schlaufe strömenden Reaktionsgemisch zusätzlicher Wasserstoff, bevorzugt möglichst nahe am Reaktor, zugeführt, um die Mindestkonzentration des Wasserstoffs in der externen Schlaufe, d. h. im Umpumpstrom, sicherzustellen.

In einer weiteren bevorzugten Ausführungsform des erfindungsgemäßen Verfahrens wird dem Reaktor an beliebiger Stelle Wasserstoff zugeführt und der Reaktordurchmesser oder die Abströmgeschwindigkeit des Reaktionsgemisches aus dem Reaktorsumpf werden so ausgelegt, dass die Mindestkonzentration des Wasserstoffs in der externen Schlaufe sichergestellt ist. Die Abströmgeschwindigkeit des Reaktionsgemisches kann durch dem Fachmann bekannte Maßnahmen, beispielsweise eine geeignete Gestaltung des Reaktordurchmessers, beeinflusst werden.

Um den externen Wälzkreis antreiben zu können, wird bevorzugt eine Pumpe installiert, die neben Flüssigkeit auch Gas und suspendierten Feststoff fördern kann. Dabei sollten bis zu 20 Vol.-% Gas und 20 Gew.-%. suspendierter Feststoff pumpbar sein.

Die Reaktionstemperatur des erfindungsgemäßen Verfahrens beträgt im Allgemeinen 50 bis 250 °C, bevorzugt 80 bis 200 °C, besonders bevorzugt 110 bis 190 °C, beispielsweise 110 bis 130 °C oder 170 bis 190 °C.

Der Reaktionsdruck des erfindungsgemäßen Verfahrens beträgt im Allgemeinen 5 bis 50 bar absolut, bevorzugt 10 bis 50 bar absolut, besonders bevorzugt 10 bis 40 bar absolut, insbesondere bevorzugt 20 bis 35 bar absolut.

### Figuren

Figur 1 zeigt die Einsatzmenge DNT (durchgezogene Linie, in kg/h) und eingeleitete Wasserstoffmenge (gestrichelte Linie, in Nm³/h) über der Zeit in Stunden für Beispiel 1.
Figur 2 zeigt die Einsatzmenge DNT (durchgezogene Linie, in kg/h) und eingeleitete Wasserstoffmenge (gestrichelte Linie, in Nm³/h) über der Zeit in Stunden für Beispiel 2.
Figur 3 zeigt die Einsatzmenge o-NT (durchgezogene Linie, in kg/h) und eingeleitete Wasserstoffmenge (gestrichelte Linie, in Nm³/h) über der Zeit in Stunden für Beispiel 3.
Figur 4 zeigt den Verlauf des DNT-Umsatzes in Prozent bei unterschiedlichen DNT-Mengen im Laborreaktor über der Zeit in Minuten für Beispiel 4.
Figur 5 zeigt den Verlauf des DNT-Umsatzes in Prozent bei unterschiedlichen DNT-Mengen im Laborreaktor über die Zeit in Minuten entsprechend Vergleichsbeispiel 1.

### Beispiele

### Beispiel 1

Für die katalytische Hydrierung von DNT zu TDA wurde ein zylinderförmiger Schlaufenreaktor mit einem durch zwei in Reihe geschaltete Kreiselpumpen mit doppelt wirkender Gleitringdichtung und Sperrdruckaggregat angetriebenen, in einer zentral am Reaktorkopf angeordneten Treibstrahldüse mündenden externen Kreislauf, einem konzentrischen Einsteckrohr sowie einer Prallplatte im unteren Reaktorteil zur Umlenkung der Schlaufenströmung (interner Kreislauf) eingesetzt (zum Funktionsprinzip vgl. WO2000/35852 A1). Das Reaktionsvolumen des Reaktors betrug rund 14 m³. Der Reaktor war zur Abfuhr der Reaktionswärme mit einem Rohrbündel aus parallel geschalteten Fieldrohren versehen. Er besaß einen Mantel zur Beheizung mit 2,5-barü Dampf. Die Menge des in die Fieldrohre eingespeisten Kühlwassers und/oder (während Unterbrechungen der DNT-Umsetzung sowie bei An- und Abfahrprozessen) die Menge des durch den Mantel geführten Dampfes wurde so eingestellt, dass die Reaktortemperatur im Bereich der Fieldrohre bei 115 bis 120 °C gehalten wurde.

Zur Aufrechterhaltung der Schlaufenströmung wurde im externen Produktkreislauf ein Volumenstrom von 630 m³/h umgewälzt, wodurch sich über die Treibstrahldüse ein Druckverlust von ca. 2,7 bar einstellte. Der Reaktor enthielt ca. 12 m³ eines flüssigen Hydrierbads. Dieses bestand im Wesentlichen aus einer Mischung von TDA (einschließlich Nebenprodukten) und Wasser im Massenverhältnis von 0,57 : 0,43, in welcher ca. 7 Gew.-% eines auf SiO₂ und ZrO₂ geträgerten metallischen Ni-Katalysators (hergestellt nach Beispiel 2 der EP 1 161 297 und mittels einer Rührwerksmühle zerkleinert; hierbei bestanden 10 Vol-% des Katalysators aus Partikeln mit einem Durchmesser < ca. 4 um (d10), 50 Vol-% sind < ca. 13 µm (d₅₀), und 90 Vol-% < ca. 19 µm (d₉₀), gemessen mittels Laserbeugung (Malvern Mastersizer S) nach Aufrühren in Wasser) suspendiert und außerdem Wasserstoff gelöst waren. Der Flüssigkeitsspiegel befand sich hierbei knapp unterhalb der Mündung der Treibstrahldüse. Darüber befanden sich ca. 2 m einer Gasatmosphäre, deren Wasserstoffgehalt durch kontinuierliche Ausschleusung eines geringen Abgasstroms auf ca. 95 Vol-% (neben Inertgasen wie zum Beispiel N₂) eingestellt wurde.

Dem externen Produktkreislauf wurde auf der Druckseite der zweiten Kreiselpumpe kontinuierlich eine Menge an Hydrierprodukt entnommen und in einen Lamellenklärer mit ca. 50 m³ Flüssigkeits- und ca. 10 m³ Gasvolumen geleitet. In dessen unterem Bereich konnte sich der Katalysator aufkonzentrieren. Ca. 18 m³/h einer entsprechend eingedickten Suspension wurden dann auf die Saugseite der ersten Kreiselpumpe zurückgeführt.

Während Unterbrechungen der DNT-Zufuhr betrug die dem Lamellenklärer zugeführte Hydrierproduktmenge ebenfalls ca. 18 m³/h; ansonsten wurde sie entsprechend der DNT-Zufuhr erhöht, um den Flüssigkeitsstand im Reaktor konstant zu halten. Gleichzeitig wurden dem Lamellenklärer über einen Überlauf bis zu 7,6 t/h Hydrierprodukt entnommen. Dieses enthielt ca. 56 Gew.-% TDA (mit einer Isomerenverteilung entsprechend der des eingesetzten DNT), ca. 1 Gew.-% Leicht- und Schwersieder (im Verhältnis von etwa 20 : 80) sowie ca. 43 Gew.-% Wasser und ca. 0,01 Gew.-% Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt. Die produktberührenden Teile waren teilweise aus Schwarzstahl (i.d.R. St37) und teilweise aus Edelstahl (1.4571) gefertigt.

Während der DNT-Zufuhr wurden über eine Membranpumpe kontinuierlich 0,6 t/h einer Suspension des oben genannten Katalysators in Wasser (teilweise im Aufarbeitungsteil aus dem Hydrierprodukt abgetrennt) in den Reaktor dosiert. Die Menge des enthaltenen Frischkatalysators in dieser Suspension betrug hierbei im Mittel etwa 1 kg/h. Mit einer Membrankolbendosierpumpe wurde in den Gasraum des Reaktors geschmolzenes und auf ca. 80 °C temperiertes DNT, bestehend aus einer Mischung der 2,4- und 2,6-DNT-Isomeren im Verhältnis von ca. 80 : 20, sowie ca. 5% der übrigen DNT-Isomeren und Spuren von Mononitrotoluol eingedüst. Gesteuert durch eine Druckregelung wurde gleichzeitig über einen Düsenring oberhalb der Prallplatte soviel Wasserstoff (verdünnt mit ca. 0,1 % N₂ sowie Spuren von Methan, CO und Inerten) eingeleitet, dass der Druck zwischen 24,9 und 25,1 barü praktisch konstant gehalten wurde.

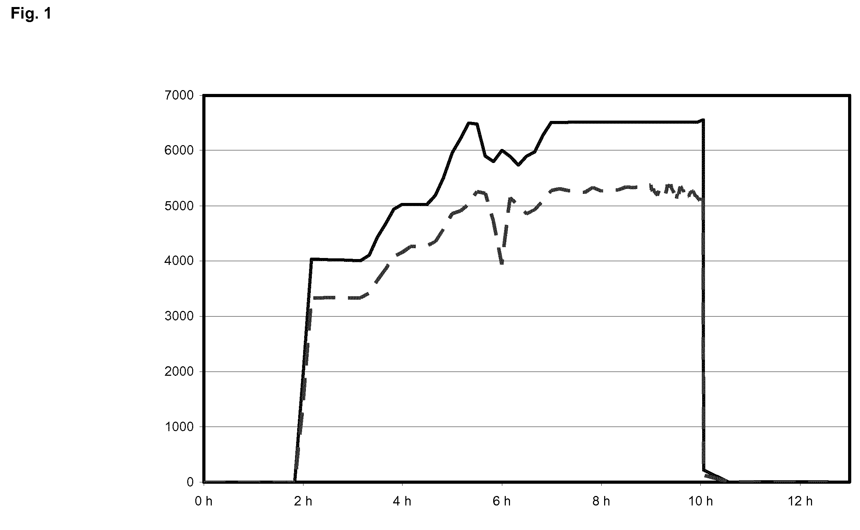

Beginnend mit 4 t/h zum Zeitpunkt 2 h wurde die DNT-Zufuhr hierbei entsprechend FIG. 1 schrittweise erhöht. Die eingeleitete Wasserstoffmenge stieg parallel hierzu an. Ein auf unvollständigen Umsatz hinweisendes kurzzeitiges Absinken der Wasserstoffmenge nach 6 h konnte durch Reduzierung der DNT-Zufuhr abgefangen werden. Begleitet wurde das Absinken der Wasserstoffmenge durch einen Rückgang der Temperatur am Reaktorausgang und einen Anstieg der Temperaturdifferenz über den externen Kreislauf von 0,0 K auf bis zu 1,7 K , entsprechend kann auf einen unvollständigen Umsatz im Reaktor und eine entsprechende Nachreaktion im externen Kreislauf geschlossen werden. Im Normalbetrieb lag die Temperatur im externen Kreislauf nahezu konstant bei ca. 126 °C. Im Reaktor lag die Temperatur am unteren Ende des Einsteckrohrs bei ca. 125 °C; über die Fieldrohre erfolgte dann eine Abkühlung auf ca. 119 °C. Nach 9 h begann die Wasserstoffaufnahme signifikant zu schwanken; nach 10 h brach sie - und damit auch der Umsatz im Reaktor - regelrecht ein. Als die Wasserstoffaufnahme (eingeleitete Wasserstoffmenge - Abgasmenge) 50 % der für die stöchiometrische Umsetzung von DNT zu TDA erforderlichen Menge unterschritt, wurde die DNT-Zufuhr erfindungsgemäß unterbrochen.

Zur Bestimmung des Gehalts an DNT und ANT (Aminonitrotoluol = teilhydriertes Zwischenprodukt) im Hydrierbad wurden nach etwa 3, 7 und 10 h aus der Leitung vom externen Produktkreislauf des Schlaufenreaktors zum Lamellenklärer Suspensionsproben entnommen. Diese wurden durch Filtration vom suspendierten Feststoff befreit und mittels Polarographie die Konzentration der enthaltenen Nitroverbindungen bestimmt. In den beiden ersten Proben betrug die DNT-Konzentration 13 bzw. 15 ppm und die ANT-Konzentration jeweils 3 ppm. Die kurz vor der Unterbrechung der DNT-Zufuhr entnommene Probe enthielt jedoch 84 ppm DNT und 3640 ppm ANT. Aufgrund der Analysendauer lag dieses Ergebnis allerdings erst ca. 1 Stunde nach der Unterbrechung der DNT-Zufuhr vor.

Aufgrund der rechtzeitigen erfindungsgemäßen Unterbrechung der DNT-Zufuhr kam es zu keiner erheblichen Schädigung des Katalysators. Nach einer zusätzlichen Zufuhr von ca. 20 kg Frischkatalysator konnte der Reaktor entsprechend Beispiel 2 wieder in Betrieb genommen und längere Zeit über stabil betrieben werden.

### Beispiel 2

Für die katalytische Hydrierung von DNT zu TDA wurde der Schlaufenreaktor aus Beispiel 1 verwendet. Die Menge des in die Fieldrohre eingespeisten Kühlwassers und/oder (während Unterbrechungen der DNT-Umsetzung sowie bei An- und Abfahrprozessen) die Menge des durch den Mantel geführten Dampfes wurde erneut so eingestellt, dass die Reaktortemperatur im Bereich der Fieldrohre bei 115 bis 120 °C gehalten wurde.

Zur Aufrechterhaltung der Schlaufenströmung wurde im externen Produktkreislauf ein Volumenstrom von 630 m³/h umgewälzt, wodurch sich über die Treibstrahldüse ein Druckverlust von ca. 2,7 bar einstellte. Der Reaktor enthielt ca. 12 m³ des flüssigen Hydrierbads aus Beispiel 1 sowie die nachdosierten 20 kg Frischkatalysator. Der Flüssigkeitsspiegel befand sich hierbei knapp unterhalb der Mündung der Treibstrahldüse. Darüber befanden sich ca. 2 m³ einer Gasatmosphäre, deren Wasserstoffgehalt durch kontinuierliche Ausschleusung eines geringen Abgasstroms auf ca. 95 Vol-% (neben Inertgasen wie zum Beispiel N₂) eingestellt wurde.

Dem externen Produktkreislauf wurde auf der Druckseite der 2. Kreiselpumpe kontinuierlich eine Menge an Hydrierprodukt entnommen und in den Lamellenklärer aus Beispiel 1 geleitet. In dessen unterem Bereich konnte sich der Katalysator aufkonzentrieren. Ca. 18 m³/h einer entsprechend eingedickten Suspension wurden dann auf die Saugseite der ersten Kreiselpumpe zurückgeführt. Während Unterbrechungen der DNT-Zufuhr betrug die dem Lamellenklärer zugeführte Hydrierproduktmenge ebenfalls ca. 18 m³/h; ansonsten wurde sie entsprechend der DNT-Zufuhr erhöht um den Flüssigkeitsstand im Reaktor konstant zu halten. Gleichzeitig wurden dem Lamellenklärer über einen Überlauf bis zu 7,3 t/h Hydrierprodukt entnommen. Dieses enthielt ca. 56 Gew.-% TDA (mit einer Isomerenverteilung entsprechend der des eingesetzten DNT), ca. 1 Gew.-% Leicht- und Schwersieder (im Verhältnis von etwa 20 : 80) sowie ca. 43 Gew.-% Wasser und ca. 0,01 Gew.-% Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt.

Während der DNT-Zufuhr wurden über eine Membranpumpe kontinuierlich 0,6 t/h einer Suspension des oben genannten Katalysators in Wasser (teilweise im Aufarbeitungsteil aus dem Hydrierprodukt abgetrennt) in den Reaktor dosiert. Die Menge des enthaltenen Frischkatalysators in dieser Suspension betrug hierbei im Mittel etwa 2 kg/h. Mit einer Membrankolbendosierpumpe wurde in den Gasraum des Reaktors geschmolzenes und auf ca. 80 °C temperiertes DNT, bestehend aus einer Mischung der 2,4- und 2,6-DNT-Isomeren im Verhältnis von ca. 80 : 20, sowie ca, 5% der übrigen DNT-Isomeren und Spuren von Mononitrotoluol eingedüst. Gesteuert durch eine Druckregelung wurde gleichzeitig über einen Düsenring oberhalb der Prallplatte soviel Wasserstoff (verdünnt mit ca. 0,1 % N₂ sowie Spuren von Methan, CO und Inerten) eingeleitet, dass der Druck zwischen 24,9 und 25,1 barü praktisch konstant gehalten wurde. Ab einer DNT-Menge von 5 t/h wurden zusätzlich 320 Nm³/h Wasserstoff am Reaktorausgang in den externen Produktkreislauf dosiert.

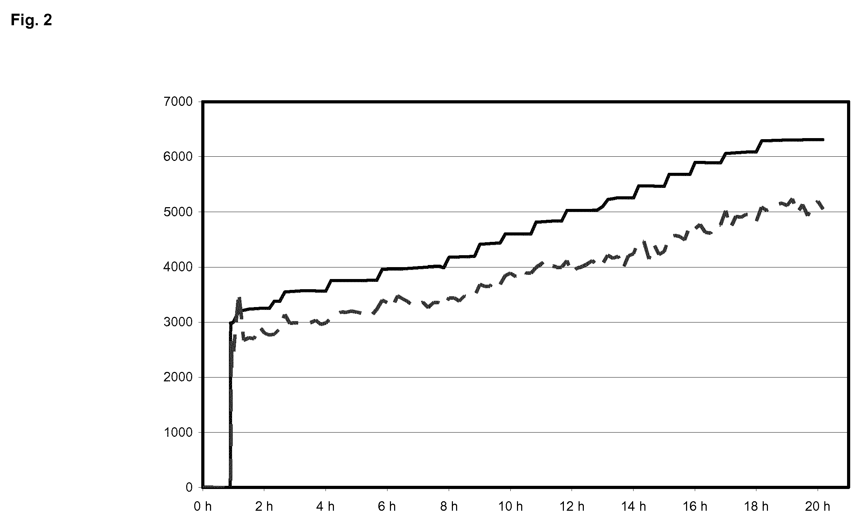

Beginnend mit 3 t/h zum Zeitpunkt 1 h wurde die DNT-Zufuhr hierbei entsprechend FIG 2 langsamer und in kleineren Schritten als im Beispiel 1 erhöht. Die eingeleitete Wasserstoffmenge stieg parallel hierzu an. Die Temperatur im externen Kreislauf lag nahezu konstant bei ca. 126 °C. Im Reaktor betrug sie am unteren Ende des Einsteckrohrs ca. 125 °C; über die Fieldrohre erfolgte dann eine Abkühlung auf ca. 119 °C.

Zur Bestimmung des Gehalts an DNT und ANT im Hydrierbad wurden nach etwa 1, 2, 3, 4, 5, 8, 12, 16 und 20 h aus der Leitung vom externen Produktkreislauf des Schlaufenreaktors zum Lamellenklärer Suspensionsproben entnommen. Diese wurden durch Filtration vom suspendierten Feststoff befreit und mittels Polarographie die Konzentration der enthaltenen Nitroverbindungen bestimmt. Die DNT-Konzentration betrug hierbei zwischen 9 und 28 ppm und die ANT-Konzentration zwischen 3 und 12 ppm. Es wurden keine Auffälligkeiten beobachtet und der Betrieb konnte über mehrere Tage hinweg problemlos fortgesetzt werden.

### Beispiel 3

Für die katalytische Hydrierung von o-Nitrotoluol zu o-Toluidin wurde ein Rührkesselreaktor (Durchmesser: 2,8 m, Höhe: 4,7 m, Volumen: 23 m³, Material: St37) mit innen im Bereich des Reaktormantels befestigten Kühlschlangen verwendet. Sein mit einem Elektromotor über Keilriemen angetriebenes Rührwerk bestand aus einem Turbinenrührer (d = 790 mm) mit Hohlwelle, der den Wasserstoff im Hydrierbad verteilte. Ein kleines Flügelrad mit 3 Flügeln (d = 250 mm) unterhalb der Turbine saugte die von dem hier verwendeten Dekantiergefäß zurückfließende eingedickte Suspension an und drückte sie nach oben in die durch die Turbine erzeugte Strömung. Die Drehzahl des Rührwerks betrug konstant 450 min⁻¹. Die Abdichtung der Welle zum Reaktor erfolgte mit einer doppeltwirkenden Gleitringdichtung mit Sperrdruckaggregat.

Die Menge des in die Kühlschlangen eingespeisten Kühlwassers wurde so eingestellt, dass die Temperatur im Reaktor bei ca. 93 °C gehalten wurde. Der Reaktor enthielt ca. 18 m³ eines flüssigen Hydrierbads. Dieses bestand im Wesentlichen aus einer Mischung von o-Toluidin, Methanol und Wasser im Massenverhältnis von 0,50 : 0,27 : 0,23, in welcher ca. 1,4 Gew.-% des in Beispiel 1 genannten, auf SiO₂ und ZrO₂ geträgerten metallischen Ni-Katalysators suspendiert und außerdem Wasserstoff gelöst waren. Oberhalb des Flüssigkeitsspiegels befanden sich ca. 5 m³ einer Gasatmosphäre, deren Wasserstoffgehalt durch kontinuierliche Ausschleusung eines geringen Abgasstroms auf 92 bis 95 Vol.-% (neben Inertgasen wie z. B. N₂) eingestellt wurde.

Um den Flüssigkeitsstand im Reaktor konstant zu halten, wurde über einen Überlauf kontinuierlich eine entsprechende Menge an Hydrierprodukt entnommen und in ein Dekantiergefäß mit ca. 16 m³ Flüssigkeits- und ca. 4 m³ Gasvolumen geleitet. In dessen unterem Bereich konnte sich der Katalysator aufkonzentrieren. Ca. 16 m³/h einer entsprechend eingedickten Suspension wurden dann mit Hilfe des Flügelrads des Rührers in den Rührkessel zurückgesaugt. Während der Hydrierung wurden dem Dekantiergefäß über einen Überlauf bis zu 1,7 t/h Hydrierprodukt entnommen. Dieses enthielt ca. 50 Gew.-% o-Toluidin, deutlich unter 1 Gew.-% Leicht- und Schwersieder, ca. 27 Gew.-% Methanol sowie ca. 23 Gew.-% Wasser und Spuren des Katalysators (v.a. Feinanteil). Das Hydrierprodukt gelangte über eine Druckreduzierung in einen Zwischenbehälter und wurde von diesem kontinuierlich der destillativen Aufarbeitung zugeführt.

Während der o-Nitrotoluol-Zufuhr wurden über eine Membranpumpe kontinuierlich 0,6 m³/h einer Suspension des oben genannten Katalysators in Methanol (teilweise im Aufarbeitungsteil aus dem Hydrierprodukt abgetrennt und noch Restwasser enthaltend) in den Reaktor dosiert. Die Menge des enthaltenen Frischkatalysators in dieser Suspension betrug hierbei im Mittel etwa 0,2 kg/h. Mit einer Membrankolbendosierpumpe wurde in den Gasraum des Reaktors flüssiges o-Nitrotoluol eingeleitet. Gesteuert durch eine Druckregelung wurde gleichzeitig soviel Wasserstoff (verdünnt mit ca. 0,1 % N₂ sowie Spuren von Methan, CO und Inerten) eingeleitet, dass der Druck zwischen 22,5 und 23,5 barü praktisch konstant gehalten wurde.

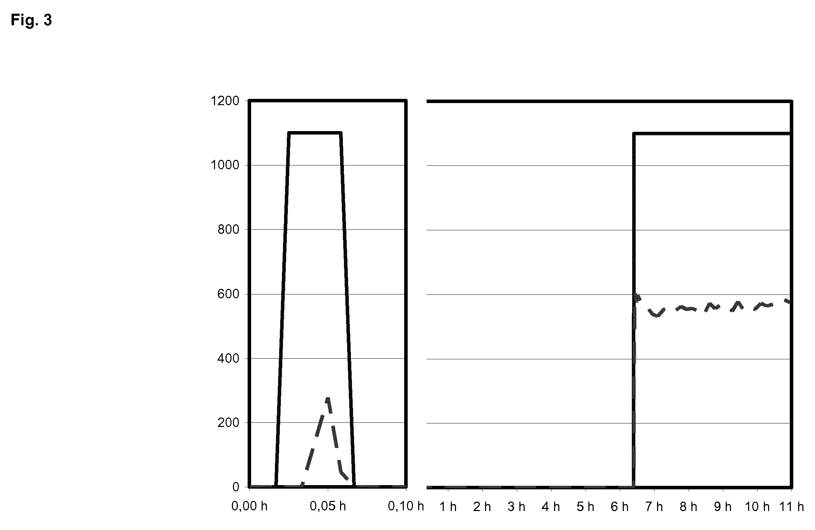

Der Verlauf der zugeführten o-Nitrotoluolmenge ist ebenso wie der der eingeleiteten Wasserstoffmenge FIG 3 zu entnehmen: Bei einem ersten Anfahrversuch wurden zu Beginn für ca. 3 min 1100 kg/h o-Nitrotoluol dosiert. Da die Wasserstoffaufnahme (eingeleitete Wasserstoffmenge - Abgasmenge) aufgrund von Verschmutzungen in der Wasserstoffleitung innerhalb dieser Zeit nicht 50% der für die stöchiometrische Umsetzung von o-Nitrotoluol zu o-Toluidin erforderlichen Menge erreichte, wurde die o-Nitrotoluol-Dosierung dann erfindungsgemäß unterbrochen. Nach Beseitigung der Verschmutzungen erfolgte ca. 6 Stunden später ein weiterer Anfahrversuch mit 1100 kg/h o-Nitrotoluol, bei dem die Wasserstoffaufnahme zügig die für die stöchiometrische Umsetzung von o-Nitrotoluol zu o-Toluidin erforderliche Menge von ca. 570 Nm³/h erreichte (vgl. FIG 3). Die Reaktion verlief unter weitgehend isothermen Bedingungen.

Zur Bestimmung des o-Nitrotoluolgehalts im Hydrierbad wurden zunächst im Abstand von 30 min und später im Abstand von 4 h aus der Leitung vom Rührkesselreaktor zum Dekantiergefäß Suspensionsproben entnommen. Diese wurden durch Filtration vom suspendierten Feststoff befreit und mittels Polarographie der o-Nitrotoluolgehalts bestimmt. Dieser betrug zu keinem Zeitpunkt mehr als 3 ppm. Es wurden keine Auffälligkeiten beobachtet und der Betrieb konnte über mehrere Wochen hinweg problemlos fortgesetzt werden.

### Beispiel 4

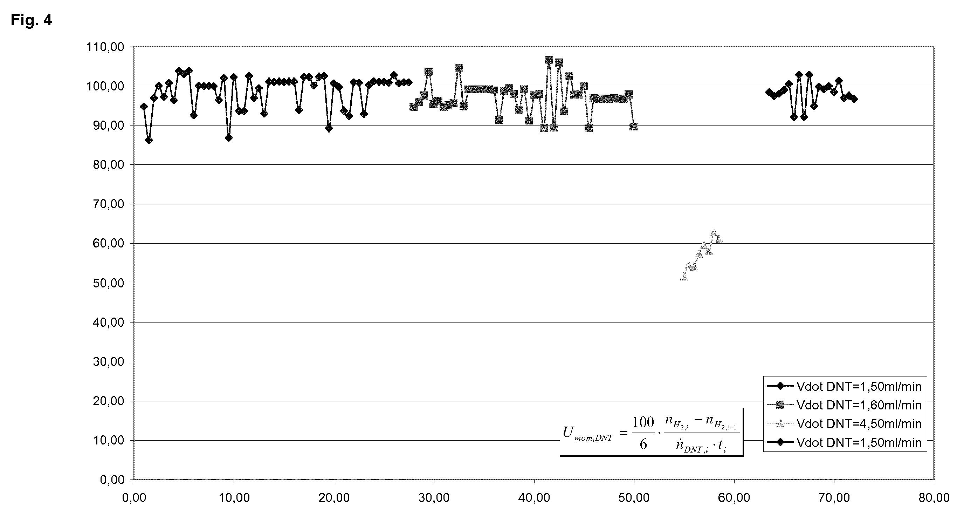

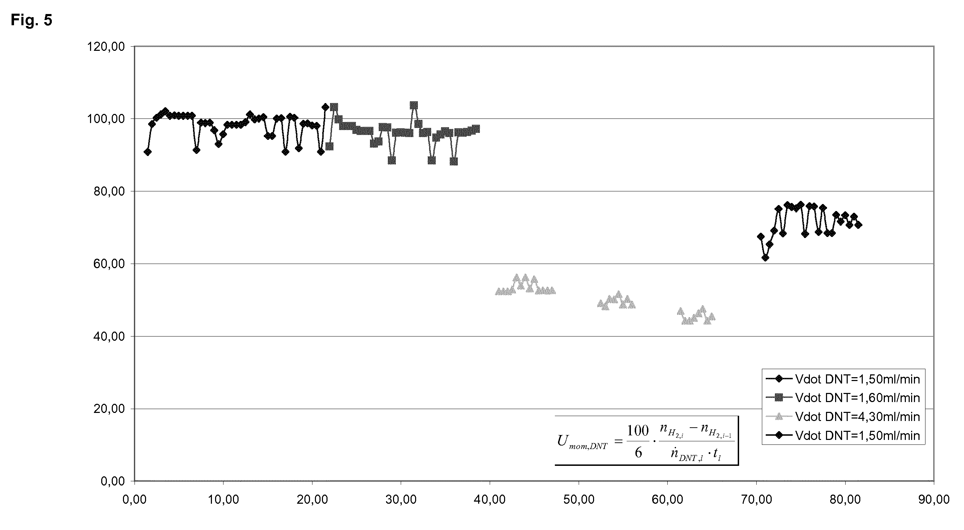

Für die katalytische Hydrierung von DNT zu TDA wurde ein mantelbeheizter Rührkesselreaktor (Volumen: 2,2 I, Material: 1.4571) mit innen liegender Kühlschlange und Scheibenrührer verwendet. Der Reaktor enthielt 1,3 I Wasser, in welchem 0,2 g des Ni-Katalysators aus Beispiel 1 (abweichend hiervon waren 50 Vol% < ca. 6 um (d₅₀)) suspendiert waren und ferner Wasserstoff gelöst sowie eine Wasserstoffatmosphäre oberhalb des Flüssigkeitsspiegels. Der Reaktor wurde bei 130 °C temperaturgeregelt und einer Rührerdrehzahl von 1500 min⁻¹ in halbkontinuierlicher (semibatch) Fahrweise ohne Ausschleusung betrieben. Mit einer HPLC-Pumpe (Kolbendosierpumpe) wurden unterschiedliche Mengen an geschmolzenem und auf ca. 80 °C temperiertem DNT in den Reaktor dosiert. Gesteuert durch eine Druckregelung wurde gleichzeitig soviel Wasserstoff eingeleitet, dass der Druck bei ca. 25 barü praktisch konstant gehalten wurde. Die Mengen an auf diese Weise mittels Wasserstoffdosiereinheit zugeführtem Wasserstoff und mit HPLC-Pumpe dosiertem DNT wurden kontinuierlich überwacht und anhand der Abweichung der für die stöchiometrische Umsetzung von DNT zu TDA erforderlichen Wasserstoffmenge eine Menge akkumuliertes DNT berechnet. Abbruchkriterium für die DNT-Dosierung war aus Sicherheitsgründen ein freier DNT-Gehalt von 1 Gew.-% (Absinken der Onset-Temperatur der Reaktionsmischung bei steigender DNT-Konzentration). Ein erneuter Start der DNT-Dosierung war dann nur nach dem Abreagieren des akkumulierten DNTs möglich. Hierzu blieb die Wasserstoffzufuhr in Betrieb. Somit konnte nicht umgesetztes DNT mit Wasserstoff zum Amin abreagieren. Hierdurch ergibt sich der in FIG 4 und FIG 5 erkennbare zeitliche Versatz (Abstand) bis zum erneuten Starten der DNT-Dosierung.

Nach einer Katalysatoraktivierung von 30 min bei 130 °C und 25 barü wurde die DNT-Dosierung wie in FIG 4 abgebildet zunächst unter Standardbedingungen (1,5 ml/min) gestartet. Hierbei wurde ein Umsatz von 100% (Wasserstoffaufnahme entsprechend der für die stöchiometrische Umsetzung des DNT zu TDA erforderlichen Menge) erreicht. Die mit der HPLC-Pumpe in den Reaktor dosierte DNT-Menge wurde dann zunächst stufenweise von 1,5 auf bis zu 4,5 ml/min gesteigert. Bei dieser Maximallast wurde nur noch ein Umsatz von 50 - 60% erreicht. Nach 5 min bei diesem geringen Umsatz wurde die DNT-Dosierung erfindungsgemäß beendet. Die anschließende Wiederholung der DNT-Dosierung unter Standardbedingungen (1,5 ml/min) führte erneut zu einem Umsatz von 100% und zeigt damit, dass durch die erfindungsgemäße schnelle Beendigung der DNT-Dosierung eine Schädigung des Katalysators verhindert werden konnte.

### Vergleichsbeispiel 1

Der Laborversuch aus Beispiel 4 wurde wiederholt, allerdings erfolgte die DNT-Dosierung bei Maximallast (hier 4,3 ml/min) in diesem Fall für insgesamt 15 min darunter 10 min lang bei einem Umsatz ≤ 50% (Wasserstoffaufnahme < 50% der für die stöchiometrische Umsetzung des DNT zu TDA erforderlichen Menge) - also deutlich länger als erfindungsgemäß zulässig. Die anschließende Wiederholung der DNT-Dosierung unter Standardbedingungen (1,5 ml/min) führte in diesem Fall nur noch zu einem Umsatz von ca. 75% (siehe FIG 5). D. h., dass während der 10-minütigen DNT-Dosierung bei einem Umsatz < 50% eine irreversible Schädigung des Katalysators erfolgte - vermutlich eine oxidative Vergiftung durch nitroaromatische Verbindungen (DNT oder nitroaromatische Zwischenprodukte wie z.B. ANT).

## Patentansprüche

1. Kontinuierliches Verfahren zur Herstellung von mindestens einem aromatischen Amin durch Hydrierung von mindestens einem Nitroaromaten mit Wasserstoff, wobei eine mindestens das aromatische Amin enthaltende Flüssigphase und eine mindestens Wasserstoff enthaltende Gasphase vorliegen, in Gegenwart eines in der flüssigen Phase suspendierten Katalysators bei einer Temperatur von 50 bis 250 °C und einem Druck von 5 bis 50 bar, **dadurch gekennzeichnet, dass**
- der Druck im Reaktor durch kontinuierliche Anpassung der Menge des dem Reaktor zugeführten Wasserstoffs so gehalten wird, dass er höchstens 10 % um den gewünschten Reaktordruck nach oben oder unten schwankt,
- die dem Reaktor insgesamt zugeführte Menge an Wasserstoff überwacht wird und
- die Zufuhr des mindestens einen Nitroaromaten unterbrochen wird, sofern die Wasserstoffaufnahme des Reaktors nicht mindestens 50 Mol-%, bevorzugt mindestens 70 Mol-%, besonders bevorzugt mindestens 90 Mol-%, ganz besonders bevorzugt mindestens 95 Mol-%, insbesondere mindestens 98 Mol-%, der für die stöchiometrische Umsetzung des mindestens einen Nitroaromaten zu dem mindestens einen aromatischen Amin benötigten Wasserstoffmenge beträgt.

2. Verfahren nach Anspruch 1, **dadurch gekennzeichnet, dass** die der stöchiometrischen Umsetzung des mindestens einen Nitroaromaten entsprechende Wasserstoffaufnahme des Reaktors spätestens 5 min., bevorzugt spätestens 3 min., nach dem Start der Dosierung des Nitroaromaten erreicht ist.

3. Verfahren nach Anspruch 1 oder 2, **dadurch gekennzeichnet, dass** Toluylendiamin aus Dinitrotoluol oder Toluidin aus Nitrotoluol oder Anilin aus Nitrobenzol hergestellt wird.

4. Verfahren nach einem der Ansprüche 1 bis 3, **dadurch gekennzeichnet, dass** die im Normalbetrieb kontinuierlich zugeführte Menge an mindestens einem Nitroaromaten durch schrittweise oder kontinuierliche Zunahme 0,5 bis 120 Stunden nach Start des Verfahrens erreicht wird.

5. Verfahren nach einem der Ansprüche 1 bis 4, **dadurch gekennzeichnet, dass** die Flüssigphase ein Lösungsmittel enthält, ausgewählt aus der Gruppe bestehend aus Wasser, organischen Lösungsmitteln, mindestens einem aromatischen Amin, welches während der erfindungsgemäßen Umsetzung gebildet wird, und Mischungen davon.

6. Verfahren nach einem der Ansprüche 1 bis 5, **dadurch gekennzeichnet, dass** die Flüssigphase zumindest teilweise aus dem Hydrierbad eines anderen Reaktors, bevorzugt aus einem Reaktor, der auch für die Umsetzung des gleichen Nitroaromaten mit Wasserstoff zu dem entsprechenden aromatischen Amin verwendet wird, und/oder einem Produktstrom vor, während oder nach der Aufarbeitung eines solchen, stammt.

7. Verfahren nach einem der Ansprüche 1 bis 6, **dadurch gekennzeichnet, dass** die Wasserstoffkonzentration in der Gasphase des Reaktors 80 bis 99 Vol.-%, bevorzugt 85 bis 98 Vol.-%, besonders bevorzugt 90 bis 95 Vol.-%, beträgt.

8. Verfahren nach einem der Ansprüche 1 bis 7, **dadurch gekennzeichnet, dass** die Zufuhr des mindestens einen Nitroaromaten mit 10 bis 90 Mol.-%, bevorzugt 20 bis 80 Mol.-%, besonders bevorzugt 30 bis 70 Mol.-%, der im Normalbetrieb zugeführten Nitroaromatenmenge begonnen wird.

9. Verfahren nach Anspruch 8, **dadurch gekennzeichnet, dass** die Nitroaromatenmenge während der Hydrierung schrittweise oder kontinuierlich erhöht wird.

10. Verfahren nach einem der Ansprüche 1 bis 9, **dadurch gekennzeichnet, dass** die Konzentration an Nitroverbindungen auf 1 bis 200 ppm eingestellt wird.

## Claims

1. A continuous process for preparing at least one aromatic amine by hydrogenation of at least one nitroaromatic by means of hydrogen, where a liquid phase comprising at least the aromatic amine and a gas phase comprising at least hydrogen are present, in the presence of a catalyst suspended in the liquid phase at a temperature of from 50 to 250°C and a pressure of from 5 to 50 bar, wherein
- the pressure in the reactor is kept so that it fluctuates by not more than 10% above or below the desired reactor pressure by continuous adaptation of the amount of hydrogen fed to the reactor,
- the total amount of hydrogen fed to the reactor is monitored and
- the introduction of the at least one nitroaromatic is interrupted if the hydrogen uptake in the reactor is not at least 50 mol%, preferably at least 70 mol%, particularly preferably at least 90 mol%, very particularly preferably at least 95 mol%, in particular at least 98 mol%, of the amount of hydrogen required for stoichiometric reaction of the at least one nitroaromatic to form the at least one aromatic amine.

2. The process according to claim 1, wherein the hydrogen uptake in the reactor corresponding to the stoichiometric reaction of the at least one nitroaromatic is reached not more than 5 minutes, preferably not more than 3 minutes, after the commencement of the introduction of the nitroaromatic.

3. The process according to claim 1 or 2, wherein toluenediamine is prepared from dinitrotoluene or toluidene is prepared from nitrotoluene or aniline is prepared from nitrobenzene.

4. The process according to any of claims 1 to 3, wherein the amount of at least one nitroaromatic introduced continuously during normal operation is reached by stepwise or continuous increase from 0.5 to 120 hours after the start of the process.

5. The process according to any of claims 1 to 4, wherein the liquid phase comprises a solvent selected from the group consisting of water, organic solvents, at least one aromatic amine formed during the reaction according to the invention and mixtures thereof.

6. The process according to any of claims 1 to 5, wherein the liquid phase comes at least partly from the hydrogenation bath of another reactor, preferably from a reactor which is also used for the reaction of the same nitroaromatic with hydrogen to form the corresponding aromatic amine, and/or a product stream before, during or after the work-up of such a stream.

7. The process according to any of claims 1 to 6, wherein the hydrogen concentration in the gas phase of the reactor is from 80 to 99% by volume, preferably from 85 to 98% by volume, particularly preferably from 90 to 95% by volume.

8. The process according to any of claims 1 to 7, wherein the introduction of the at least one nitroaromatic is commenced with from 10 to 90 mol%, preferably from 20 to 80 mol%, particularly preferably from 30 to 70 mol%, of the amount of nitroaromatic introduced in normal operation.

9. The process according to claim 8, wherein the amount of nitroaromatic is increased stepwise or continuously during the hydrogenation.

10. The process according to any of claims 1 to 9, wherein the concentration of nitro compounds is set to from 1 to 200 ppm.

## Revendications

1. Procédé continu pour la préparation d'au moins une amine aromatique par hydrogénation d'au moins un nitroaromatique avec de l'hydrogène, une phase liquide contenant au moins l'amine aromatique et une phase gazeuse contenant au moins de l'hydrogène étant présentes, en présence d'un catalyseur en suspension dans la phase liquide à une température de 50 à 250°C et à une pression de 5 à 50 bars, **caractérisé en ce que**
- la pression est maintenue dans le réacteur, par adaptation continue de la quantité d'hydrogène introduite dans le réacteur, de manière telle qu'elle varie vers le haut et vers le bas d'au plus 10% autour de la pression souhaitée dans le réacteur,
- la quantité d'hydrogène totale alimentée dans le réacteur est surveillée et
- l'introduction dudit au moins un nitroaromatique est interrompue lorsque l'absorption d'hydrogène du réacteur n'est pas d'au moins 50% en mole, de préférence d'au moins 70% en mole, de manière particulièrement préférée d'au moins 90% en mole, de manière tout particulièrement préférée d'au moins 95% en mole, en particulier d'au moins 98% en mole, de la quantité d'hydrogène nécessaire pour la transformation stoechiométrique dudit au moins un nitroaromatique en ladite au moins une amine aromatique.

2. Procédé selon la revendication 1, **caractérisé en ce que** l'absorption d'hydrogène correspondant à la transformation stoechiométrique dudit au moins un nitroaromatique du réacteur est atteinte au plus tard 5 minutes, de préférence au plus tard 3 minutes, après le début du dosage du nitroaromatique.

3. Procédé selon la revendication 1 ou 2, **caractérisé en ce qu'**on prépare de la toluylènediamine à partir de dinitrotoluène ou de la toluidine à partir de nitrotoluène ou de l'aniline à partir de nitrobenzène.

4. Procédé selon l'une quelconque des revendications 1 à 3, **caractérisé en ce que** la quantité d'au moins un nitroaromatique alimentée en continu lors du fonctionnement normal est atteinte par augmentation par étapes ou continue, 0,5 à 120 heures après le démarrage du procédé.

5. Procédé selon l'une quelconque des revendications 1 à 4, **caractérisé en ce que** la phase liquide contient un solvant, choisi dans le groupe constitué par l'eau, des solvants organiques, au moins une amine aromatique, qui est formée pendant la transformation selon l'invention, et des mélanges de ceux-ci.

6. Procédé selon l'une quelconque des revendications 1 à 5, **caractérisé en ce que** la phase liquide provient au moins partiellement du bain d'hydrogénation d'un autre réacteur, de préférence d'un réacteur qui est également utilisé pour la transformation du même nitroaromatique avec de l'hydrogène en l'amine aromatique correspondante, et/ou d'un flux de produits avant, pendant ou après le traitement de celui-ci.

7. Procédé selon l'une quelconque des revendications 1 à 6, **caractérisé en ce que** la concentration en hydrogène dans la phase gazeuse du réacteur est de 80 à 99% en volume, de préférence de 85 à 98% en volume, de manière particulièrement préférée de 90 à 95% en volume.

8. Procédé selon l'une quelconque des revendications 1 à 7, **caractérisé en ce que** l'introduction dudit au moins un nitroaromatique est démarrée par 10 à 90% en mole, de préférence par 20 à 80% en mole, de manière particulièrement préférée par 30 à 70% en mole, de la quantité de nitroaromatique introduite lors du fonctionnement normal.

9. Procédé selon la revendication 8, **caractérisé en ce que** la quantité de nitroaromatique est augmentée par étapes ou en continu pendant l'hydrogénation.

10. Procédé selon l'une quelconque des revendications 1 à 9, **caractérisé en ce que** la concentration en composés nitro est réglée à 1 jusqu'à 200 ppm.
